# FASCICULE DE BREVET EUROPEEN

(11) **EP 4 580 546 B1**
(45) Date de publication et mention de la délivrance du brevet: **07.10.2026**
(21) Numéro de dépôt: 23762424.2
(22) Date de dépôt: 29.08.2023
(51) Int. Cl.: A61C 9/00, G06V 20/20, H04N 23/60

(54) **PROCÉDÉ DE SCANNAGE D'UNE ARCADE DENTAIRE D'UN UTILISATEUR**
VERFAHREN ZUM SCANNEN EINES ZAHNBOGENS EINES BENUTZERS
METHOD FOR SCANNING A DENTAL ARCH OF A USER

(30) Priorité: 31.08.2022 FR 2208709
(43) Date de publication de la demande: 09.07.2025
(73) Titulaire: Dental Monitoring, 75017 Paris (FR)
(72) Inventeur: PELLISSARD, Thomas, 94700 Maisons-Alfort (FR); GHYSELINCK, Guillaume, 59169 CANTIN (FR); DOREMUS, Xavier, 92120 MONTROUGE (FR)
(74) Mandataire: Novagraaf Group
(86) Numéro de dépôt international: PCT/EP2023/073628
(87) Numéro de publication internationale: WO 2024/047019

(56) Documents cités:
- US-A1- 2018 060 690
- US-A1- 2020 193 871

## Description

### Domaine technique

La présente invention concerne un procédé pour scanner une arcade dentaire d'un utilisateur, et un dispositif pour mettre en œuvre un tel procédé.

### Technique antérieure

Il est classique d'effectuer un scan des arcades dentaires d'un utilisateur afin d'analyser sa situation dentaire, en particulier avant d'établir un traitement orthodontique. Cependant, la réalisation d'un scan des arcades dentaires d'un utilisateur nécessite que l'utilisateur se déplace chez un professionnel de soins dentaires ou qu'il transmette des informations, en particulier des images, au professionnel de soins dentaires. La réalisation d'un scan au sein d'un cabinet dentaire ou plus généralement chez un professionnel de soins dentaires peut cependant engendrer des coûts importants mais également un stress pour l'utilisateur. De plus, l'acquisition d'images dentaires par l'utilisateur lui-même peut amener à une qualité d'images insuffisante ou à un contenu incomplet.

D1 divulgue un dispositif et une méthode d'apprentissage semi-automatique du scannage d'une arcade dentaire, où l'apprenant est averti lorsqu'il dévie de la trajectoire idéale.

Il existe un besoin pour faciliter l'acquisition d'un scan d'arcades dentaires, en limitant le risque d'un scan incomplet.

Un but de l'invention est de répondre à ce besoin.

### Exposé de l'invention

### Résumé de l'invention

L'invention propose un procédé de scannage d'une arcade dentaire d'un utilisateur, le procédé comportant
- une étape préalable de détermination d'une représentation d'une arcade dentaire de référence, dite « représentation de référence », puis
- une pluralité de cycles d'étapes a) à d) suivantes :
   a) acquisition, dans des conditions d'acquisition, d'une image bidimensionnelle de ladite arcade dentaire, ou « image actualisée », au moyen d'un appareil d'acquisition d'images, de préférence par l'utilisateur ;
   b) analyse de l'image actualisée de manière à identifier la surface de l'image actualisée représentant l'arcade dentaire de l'utilisateur, dite « surface 2D acquise », puis délimitation et marquage d'une surface de la représentation de référence correspondant à la surface 2D acquise, ou « zone marquée élémentaire », et ajout de la zone marquée élémentaire dans une « zone marquée de la représentation de référence » regroupant l'ensemble, vide avant le premier dit cycle, des zones marquées élémentaires définies lors des cycles précédents ;
   c) détermination et présentation à l'utilisateur d'une information de guidage de l'appareil d'acquisition vers de nouvelles conditions d'acquisition adaptées pour que le cycle suivant augmente la zone marquée de la représentation de référence ;
   d) déplacement de l'appareil d'acquisition vers lesdites nouvelles conditions d'acquisition ; le procédé comportant de préférence, après le dernier cycle, une étape finale de génération d'un modèle tridimensionnel final à partir desdites images actualisées.

La génération du modèle final peut être réalisée par l'appareil d'acquisition d'images ou par un ordinateur distant de l'appareil d'acquisition d'images et à qui l'ensemble des images actualisées acquises a été transmis, de préférence à un ordinateur d'un professionnel des soins dentaires.

Comme on le verra plus en détail dans la suite de la description, un procédé de scannage selon l'invention conduit rapidement et facilement à un ensemble d'image actualisées suffisant pour fabriquer un modèle final complet de l'arcade dentaire.

En outre, il n'est pas nécessaire que l'utilisateur se déplace chez un professionnel de soins dentaires, ni que l'utilisateur soit supervisé par un professionnel de soins dentaires pour acquérir les images actualisées. Grâce au guidage, les images actualisées peuvent avantageusement être acquises dans des conditions d'acquisition précises, sans formation particulière.

Avantageusement, l'acquisition peut s'effectuer en temps réel, les images actualisées étant par exemple extraites d'un film.

De préférence, à l'étape c), l'information de guidage est présentée sur un écran, de préférence sur un écran de l'appareil d'acquisition d'images. L'appareil d'acquisition d'images peut être en particulier un téléphone portable ou une tablette, de préférence un téléphone portable.

L'information de guidage peut en particulier comporter une information sonore, une information visuelle, et/ou une information sensitive. Ainsi, l'information de guidage peut être adaptée à tout utilisateur. De préférence l'information de guidage comporte plusieurs types d'informations différents stimulant de préférence plusieurs sens différents, facilitant ainsi la communication vers l'utilisateur.

Dans un mode de réalisation préféré de l'invention, le marquage comporte, pour chaque étape b), un marquage visuel de la surface de la représentation de référence, et chaque étape c) comporte une présentation, de préférence à l'utilisateur, de préférence sur un écran, de préférence sur un écran d'un téléphone portable de l'utilisateur ayant servi à l'acquisition des images actualisées, de l'ensemble des marques visuelles générées à l'ensemble des étapes b) précédentes.

Une marque visuelle comporte de préférence une représentation graphique de la zone marquée élémentaire, par exemple une représentation du contour de la zone élémentaire marquée et/ou un aplat d'une couleur et/ou d'un motif dans les limites de la zone élémentaire marquée.

L'acquisition en est alors simplifiée. En particulier, lors d'un cyclage en temps réel, l'écran affiche une représentation de référence dont la zone marquée se complète au fur et à mesure des cycles, ce qui permet à l'utilisateur d'identifier facilement la zone non marquée, et de positionner et orienter l'appareil d'acquisition d'images en conséquence.

Réciproquement, lors d'un cyclage en temps réel, l'écran peut afficher une représentation de référence dont la zone non marquée s'efface au fur et à mesure des cycles. De préférence, l'écran continue cependant à représenter au moins les contours de l'arcade dentaire.

Les marques visuelles constituent ainsi une information de guidage préférée.

L'information de guidage peut comporter des informations annexes, les informations annexes correspondant aux informations autres que celles destinées à indiquer un positionnement de l'appareil d'acquisition, les informations annexes peuvent par exemple indiquer à l'utilisateur de mettre ou d'enlever une gouttière orthodontique, d'ouvrir ou de fermer la bouche, et/ou d'utiliser un écarteur ou au contraire de l'enlever.

La représentation de référence peut être générée. Alternativement, elle peut être sélectionnée, par exemple dans une base de données.

La représentation de référence peut être bidimensionnelle. Dans un mode de réalisation préféré, la représentation de référence est un modèle tridimensionnel numérique. Le procédé permet, en acquérant différentes images actualisées, de marquer différentes zones de ce modèle jusqu'à atteindre un seuil de couverture du modèle ou de préférence jusqu'à le marquer complètement. Les images actualisées « couvrent » alors avantageusement une majorité de la surface du modèle, ou de préférence toute la surface du modèle et permettent, par exemple de fabriquer un modèle final représentant avec un haute précision l'arcade dentaire de l'utilisateur. Le modèle final peut être réalisé par un ordinateur du professionnel de soins dentaires à qui les images actualisées sont transmises, ou réalisé par l'appareil d'acquisition d'images.

En particulier, le seuil de couverture peut être de 90%, mieux de 95% encore mieux de 99%.

Le procédé peut comporter à la fin de chaque cycle, la comparaison du pourcentage de la surface de la zone marquée par rapport à la surface totale du modèle, appelé « pourcentage de couverture » avec le seuil de couverture. Si le pourcentage de couverture est supérieur au seuil alors on effectue l'étape finale de génération d'un modèle, à l'inverse on reprend un nouveau cycle.

Dans un mode de réalisation particulier, à l'étape c), le pourcentage de couverture est présenté à l'utilisateur.

La présentation du pourcentage de couverture peut prendre la forme d'une jauge, d'un affichage numérique, d'une barre de progression, d'objectifs présentés successivement lorsqu'un objectif particulier est atteint ou simultanément et présentant un affichage différent distinguant les objectifs atteints de ceux qu'il reste à atteindre. Par exemple les objectifs sont « acquisition des molaires » , « acquisition des incisives », « acquisition de plus de 10% du modèle », « acquisition de plus de 25% du modèle », « acquisition de plus de 40% du modèle » , « acquisition de plus de 50% du modèle », « acquisition de plus de 75% du modèle », « acquisition de plus de 85% du modèle », « acquisition de vue de droite », « acquisition de vue de gauche », « acquisition de vue de haut », « acquisition de vue de bas » ; « acquisition d'une vue bouche fermée », « acquisition d'une vue bouche ouverte», « acquisition avec une gouttière orthodontique ».

Un procédé selon l'invention peut encore notamment comporter une ou plusieurs des caractéristiques optionnelles suivantes :
- les images actualisées des cycles successifs sont extraites d'un film acquis par l'utilisateur par une tablette ou un téléphone portable, lesdits cycles étant de préférence exécutés en temps réel pendant l'acquisition dudit film, au moins une partie de l'information de guidage étant de préférence présentée sur un écran de ladite tablette ou dudit téléphone, respectivement ;
- l'information de guidage comporte une présentation de la zone marquée sur un écran de l'appareil d'acquisition d'images, de préférence en superposition avec la représentation de référence dans laquelle la zone non marquée présente une apparence, de préférence une couleur, différente de la zone marquée ;
- la représentation de référence est un modèle « de référence » d'une arcade « de référence »
   - représentative des arcades « historiques » d'un ensemble comportant plus de 10, plus de 100, plus de 1 000, plus de 10 000 et/ou moins de 1 000 000 individus historiques, ou
   - constituée par assemblage de modèles de dents suivant un agencement déterminé par analyse d'une ou plusieurs images d'analyse de l'arcade dentaire de l'utilisateur, de préférence acquises avec l'appareil d'acquisition d'images
- une ou plusieurs illustrations, en 3D ou 2D, d'une arcade dentaire, éventuellement photoréaliste ;
- à l'étape c) d'un cycle quelconque, on présente une vue du modèle de référence observé suivant une direction d'observation orientée, par rapport au modèle de référence, comme la direction d'acquisition de l'image actualisée à l'étape a) dudit cycle, par rapport à l'arcade dentaire de l'utilisateur, c'est-à-dire sensiblement orientée comme la direction de l'axe optique de la caméra ou de l'appareil photo lors de ladite acquisition, de sorte que ladite vue est sensiblement superposable en registre avec ladite image actualisée ;
- à une étape c), de préférence à une étape c) quelconque, l'appareil d'acquisition d'images fournit une information variable en fonction d'une différence entre les conditions d'acquisition réelles et lesdites nouvelles conditions d'acquisition, par exemple en émettant un signal sonore ou une indication visuelle, par exemple un clignotement, d'autant plus intense que lesdites différences sont petites ; l'utilisateur sait ainsi avantageusement si le mouvement de l'appareil d'acquisition d'images qu'il effectue est plus ou moins efficace pour atteindre les nouvelles conditions d'acquisition ;
- pour l'étape a) d'au moins un cycle, de préférence pour une étape a) quelconque, on écarte les lèvres et/ou les joues de l'arcade dentaire de l'utilisateur, de préférence en utilisant un écarteur, de manière à exposer à l'appareil d'acquisition d'images au moins une dent, par exemple pour exposer complètement l'extrados d'une incisive et/ou au moins partiellement l'extrados d'une molaire ;
- lors du cyclage, on acquiert
   - au moins une image actualisée prise face à l'utilisateur et/ou au moins une image actualisée prise à droite de l'utilisateur et/ou au moins une image actualisée prise à gauche de l'utilisateur, de préférence au moins une image actualisée prise face à l'utilisateur et au moins une image actualisée prise à droite de l'utilisateur et au moins une image actualisée prise à gauche de l'utilisateur; et/ou
   - au moins une image actualisée prise face à l'utilisateur d'une part et au moins une image actualisée prise de haut par rapport à l'utilisateur et/ou au moins une image actualisée prise de bas par rapport à l'utilisateur, d'autre part, de préférence au moins une image actualisée prise face à l'utilisateur et au moins une image actualisée prise de haut par rapport à l'utilisateur et au moins une image actualisée prise de bas par rapport à l'utilisateur ; et/ou
   - au moins une image actualisée prise bouche ouverte et/ou au moins une image actualisée prise bouche fermée ;
- l'utilisateur est à distance, de préférence à plus de 100 m ou plus de 1 km et/ou à moins de 1000 km de tout professionnel de soins dentaires, en particulier de tout orthodontiste ;
- on arrête le cyclage quand plus de 50%, plus de 70%, plus de 90%, de préférence 100% de la surface de la représentation de référence sont « couverts » par les images actualisées acquises, c'est-à-dire sont représentés sur au moins une image actualisée ;
- on compare le modèle final à un autre modèle représentant l'arcade dentaire de l'utilisateur, par exemple pour corriger le modèle final ou l'autre modèle, et/ou on découpe le modèle final en modèles de dent et, de préférence, on déplace lesdits modèles de dent ;
- on compare le modèle final au modèle de référence, et, de préférence, on présente cette comparaison à l'utilisateur, par exemple en superposant, de préférence en registre, le modèle final et le modèle de référence ;
- la représentation de référence est un modèle tridimensionnel et on détermine la surface de la représentation de référence correspondant à la surface 2D acquise
   - en recherchant des conditions d'acquisition virtuelles adaptées pour une acquisition d'une vue de la représentation de référence, dite « image de référence », présentant une concordance maximale avec l'image actualisée dans lesdites conditions d'acquisition virtuelles ; puis
   - en projetant la surface 2D acquise sur la représentation de référence, suivant la direction d'observation de ladite représentation de référence dans lesdites conditions d'acquisition virtuelles ;
- les conditions d'acquisition virtuelles sont déterminées en cherchant à superposer des points remarquables de l'arcade dentaire représentée sur l'image actualisée avec les points remarquables correspondants de la représentation de référence.

L'invention concerne également :
- un programme d'ordinateur, et en particulier un logiciel spécialisé pour téléphone portable ou tablette, ou « appli », comprenant des instructions de code de programme pour l'exécution d'une ou plusieurs étapes d'un procédé selon l'invention, de préférence pour l'ensemble des étapes b) à c) lorsque ledit programme est exécuté par un ordinateur,
- un support informatique sur lequel est enregistré un tel programme, par exemple une mémoire ou un CD-ROM.

De préférence, lorsque le programme d'ordinateur est un logiciel spécialisé pour téléphone portable ou tablette, le programme d'ordinateur comporte des instructions pour l'exécution de l'étape a), de préférence encore pour l'exécution de l'ensemble des étapes a) à c).

L'invention concerne également un dispositif pour la mise en œuvre un procédé selon l'invention, ledit dispositif comportant :
- optionnellement, un premier ordinateur comportant un premier programme d'ordinateur comprenant des instructions de code de programme pour générer la représentation de référence ou sélectionner la représentation de référence, par exemple dans une base de données ;
- un appareil d'acquisition d'images pour la mise en œuvre des étapes a) ;
- de préférence, un écran pour la présentation d'une information de guidage lors des étapes c) ;
- un deuxième ordinateur, identique ou différent du premier ordinateur, comportant un deuxième programme d'ordinateur comprenant des instructions de code de programme pour l'exécution des étapes b) et c) ;
- un troisième ordinateur, identique ou différent du premier et/ou deuxième ordinateur(s), comportant un troisième programme d'ordinateur comprenant des instructions de code de programme pour la mise en œuvre de l'étape finale de génération du modèle final de l'arcade dentaire de l'utilisateur à partir des images actualisées ;
- de préférence, un quatrième ordinateur, identique ou différent du premier et/ou deuxième et/ou troisième ordinateur(s), comportant un quatrième programme d'ordinateur comprenant des instructions de code de programme pour comparer le modèle final à un autre modèle représentant l'arcade dentaire de l'utilisateur et/ou découper le modèle final en modèles de dent et, de préférence, déplacer lesdits modèles de dent.

De préférence, l'écran et/ou le premier ordinateur et/ou le deuxième ordinateur et/ou le troisième ordinateur et/ou le quatrième ordinateur est/sont intégré(s) dans l'appareil d'acquisition. De préférence, l'appareil d'acquisition d'images est un téléphone portable ou une tablette. De préférence, l'écran est un écran du téléphone portable ou de la tablette, respectivement.

L'utilisateur peut alors facilement, sans intervention d'un tiers, et en particulier sans intervention d'un professionnel de soins dentaires, au moyen d'un simple téléphone portable ou tablette, acquérir des images dentaires de bonne qualité et représentant ensemble entièrement la représentation de référence.

Le dispositif peut encore comporter des moyens de communication, notamment permettant d'envoyer la ou les images actualisées et/ou de recevoir la représentation de référence.

### Définitions

Un « utilisateur » est une personne pour laquelle un procédé selon l'invention est mis en œuvre.

Par « professionnel de soins dentaires », on entend toute personne qualifiée pour prodiguer des soins dentaires, ce qui inclut un orthodontiste et un dentiste.

La position « bouche fermée » est la position d'occlusion dans laquelle les dentures supérieure et inférieure de l'utilisateur sont en contact. Une position « bouche ouverte » est une position dans laquelle les dentures supérieure et inférieure de l'utilisateur ne sont pas en contact, de préférence la position d'ouverture totale de la bouche.

Par « arcade » ou « arcade dentaire », on entend tout ou partie d'une arcade dentaire, comportant de préférence au moins 2, de préférence au moins 3, de préférence au moins 4 dents.

Un « écarteur » (« retractor » en anglais), ou « écarteur dentaire », est un dispositif destiné à retrousser les lèvres. Il comporte un rebord supérieur et un rebord inférieur, et/ou un rebord droit et un rebord gauche, s'étendant autour d'une ouverture d'écarteur et destinés à être introduits entre les dents et les lèvres. En position de service, les lèvres de l'utilisateur sont en appui sur ces rebords, de sorte que les dents sont visibles à travers l'ouverture d'écarteur. Un écarteur permet ainsi d'observer les dents sans être gêné par les lèvres. Les dents ne reposent cependant pas sur l'écarteur, de sorte que l'utilisateur peut, en tournant la tête par rapport à l'écarteur, modifier les dents qui sont visibles à travers l'ouverture d'écarteur. Il peut aussi modifier l'écartement entre ses arcades dentaires. En particulier, un écarteur n'appuie pas sur les dents de manière à écarter les deux mâchoires l'une de l'autre, mais sur les lèvres. Dans un mode de réalisation, un écarteur est configuré de manière à écarter élastiquement l'une de l'autre les lèvres supérieure et inférieure de manière à dégager les dents visibles à travers l'ouverture d'écarteur. Dans un mode de réalisation, un écarteur est configuré de manière que la distance entre le rebord supérieur et le rebord inférieur, et/ou entre le rebord droit et le rebord gauche soit constante. Des écarteurs sont par exemple décrits dans PCT/EP2015/074896, US 6,923,761, ou US 2004/0209225.

Selon la convention internationale de la Fédération Dentaire Internationale, chaque dent d'une arcade dentaire a un numéro prédéterminé.

Un « point remarquable » est un point d'une arcade, d'une dent, d'un modèle d'arcade ou d'un modèle de dent que l'on peut identifier, par exemple le sommet de la dent ou à la pointe d'une cuspide, un point de contact interdentaire, c'est-à-dire d'une dent avec une dent adjacente, par exemple un point mésial ou distal du bord incisif d'une dent, ou un point au centre de la couronne de la dent, ou « barycentre ».

Par « ordinateur », on désigne une unité de traitement informatique, ce qui inclut un ensemble de plusieurs machines, ayant des capacités de traitement informatique. Cette unité peut être notamment intégrée dans un téléphone portable ou être un ordinateur de type PC ou un serveur, par exemple un serveur à distance de l'utilisateur, par exemple être le « cloud » ou un ordinateur disposé chez un professionnel des soins dentaires. Le téléphone portable et l'ordinateur comportent alors des moyens de communication pour échanger entre eux.

Classiquement, un ordinateur comporte en particulier un processeur, une mémoire, une interface homme-machine, comportant classiquement un écran, un module de communication par internet, par WIFI, par Bluetooth^{®} ou par le réseau téléphonique. Un logiciel configuré pour mettre en œuvre un procédé de l'invention est chargé dans la mémoire de l'ordinateur. L'ordinateur peut être également connecté à une imprimante.

Le procédé selon l'invention (hors les opérations d'acquisition et de déplacement de l'appareil d'acquisition d'images) est mis en œuvre par ordinateur, de préférence exclusivement par ordinateur.

Par « modèle », on entend un modèle tridimensionnel numérique. Un modèle est constitué d'un ensemble de voxels.

Un « modèle de dent » est un modèle numérique tridimensionnel d'une dent. Un modèle d'une arcade dentaire peut être découpé de manière à définir, pour au moins une partie des dents, de préférence pour toutes les dents représentées dans le modèle de l'arcade, des modèles de dent. Les modèles de dent sont donc des modèles au sein du modèle de l'arcade.

Un « modèle d'une arcade » est un modèle représentant au moins une partie d'une arcade dentaire, de préférence au moins 2, de préférence au moins 3, de préférence au moins 4 dents.

Le « découpage » d'un modèle d'une arcade en « modèles de dent » est une opération permettant de délimiter et rendre autonomes les représentations des dents (modèles de dent) dans le modèle de l'arcade. Il existe des outils informatiques pour manipuler les modèles de dent d'un modèle d'arcade. Un exemple de logiciel permettant de manipuler les modèles de dent et créer un scénario de traitement est le programme Treat, décrit sur la page *https:*//*en.wikipedia.org*/*wiki*/*Clear*_*aligners#cite*_*note-invisalignsystem-10.*

Par "image", on entend une image en deux dimensions, comme une photographie ou une image extraite d'un film. Une image est formée de pixels. Un « film » est considéré comme un ensemble de photos.

Par « image d'une arcade », ou « modèle d'une arcade », ou « représentation de référence » d'une arcade dentaire, on entend une représentation de tout ou partie de ladite arcade, représentant de préférence au moins 2 dents, de préférence au moins 4 dents.

On appelle « concordance » ou « proximité » (« match » ou « fit » en anglais) entre deux objets, par exemple entre les représentations d'une arcade dentaire sur deux images, une mesure de la différence, ou « distance », entre ces deux objets. Une concordance est maximale (« best fit ») lorsque cette différence est minimale, en particulier lorsque les deux images représentent sensiblement les mêmes éléments de la même façon, c'est-à-dire de manière que les représentations des éléments sur ces deux images soient sensiblement superposables en registre.

Une superposition de deux images est « en registre » lorsque les contours de représentations d'éléments physiques sur la première image se superposent aux contours desdits éléments physiques sur la deuxième image. La superposition apparaît ainsi réaliste.

Le « marquage » d'une surface d'une représentation de référence consiste à mémoriser informatiquement des données suffisantes reconstituer spécifiquement cette surface. Les limites de cette surface peuvent être en particulier représentées sur la représentation de référence de manière que l'utilisateur puisse la visualiser. Le marquage est alors qualifié de « visuel ».

Les « conditions d'acquisition » d'une image précisent la position et l'orientation dans l'espace d'un appareil d'acquisition de cette image relativement aux dents de l'utilisateur (conditions d'acquisition réelles) ou à un modèle de dents de l'utilisateur (conditions d'acquisition virtuelles), et de préférence la calibration de cet appareil d'acquisition. Des conditions d'acquisition sont dites "virtuelles" ou « théoriques » lorsqu'elles correspondent à une simulation dans laquelle l'appareil d'acquisition serait dans lesdites conditions d'acquisition (positionnement et de préférence calibration théoriques de l'appareil d'acquisition) par rapport à un modèle.

Notamment dans des conditions d'acquisition virtuelles d'une image de référence, l'appareil d'acquisition peut être également qualifié de « virtuel ». L'image est en effet acquise par un appareil d'acquisition fictif, ayant les caractéristiques de l'appareil d'acquisition « réel » ayant servi à l'acquisition des images réelles, et en particulier des images actualisées.

La « calibration » d'un appareil d'acquisition est constituée par l'ensemble des valeurs des paramètres de calibration. Un "paramètre de calibration" est un paramètre intrinsèque à l'appareil d'acquisition (à la différence de sa position et de son orientation) dont la valeur influence l'image acquise. De préférence, les paramètres de calibration sont choisis dans le groupe formé par l'ouverture de diaphragme, le temps d'exposition, la distance focale et la sensibilité.

Un « traitement statistique » est un traitement qui, appliqué à un ensemble de données, permet de déterminer des caractéristiques propres à cet ensemble, par exemple une moyenne, un écart-type, ou une valeur médiane. Les outils de traitement statistique sont bien connus de l'homme du métier.

Une « illustration » n'a pas de réalité physique. Cela peut être une représentation symbolique ou schématique comportant un ensemble de formes géométriques, un dessin numérique en 2D ou 3D.

« Premier », « deuxième », « troisième » et « quatrième », « de référence », « actualisé », « historique », « cible » sont utilisés à des fins de clarté.

Vertical », « horizontal », « droite », « gauche », « devant » ou « de face », « derrière », « au-dessus », « au-dessous » font référence à un utilisateur qui se tient debout, verticalement.

Il faut interpréter "comprenant " ou "comportant " ou "présentant " de manière non restrictive, sauf indication contraire.

### Brève description des dessins

D'autres caractéristiques et avantages de l'invention apparaîtront encore à la lecture de la description détaillée qui va suivre et à l'examen du dessin annexé dans lequel :
[Fig 1] la figure 1 représente schématiquement un cycle d'un procédé selon l'invention ;
[Fig 2] la figure 2 représente schématiquement un dispositif selon l'invention
[Fig 3] la figure 3 représente la coloration progressive d'un modèle de référence.

### Description détaillée

### Procédé

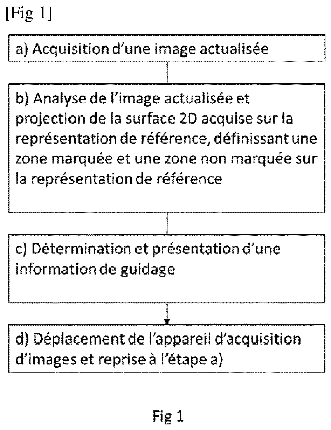

Un procédé de scannage d'une arcade dentaire d'un utilisateur selon l'invention comporte les étapes a) à d) telles qu'illustrées par la figure 1.

De préférence, il comporte également une étape préalable de détermination d'une représentation de référence.

La détermination de la représentation de référence peut consister à générer une représentation, en particulier un modèle tridimensionnel.

Dans un autre mode de réalisation, la détermination de la représentation de référence peut consister à sélectionner une représentation dans une base de données.

La représentation de référence est un modèle ou une image représentant une arcade dentaire de référence. De préférence, la représentation de référence est un modèle, dit « modèle de référence ».

La représentation de référence peut représenter une arcade dentaire de référence standard, et être utilisée pour différents utilisateurs, éventuellement quel que soit l'utilisateur. Cependant, la représentation de référence représente de préférence une arcade dentaire de référence dont la forme est proche de celle de l'utilisateur, ce qui améliore la précision du procédé.

La représentation de référence peut être représentative d'une population d'individus.

Dans un mode de réalisation, la représentation de référence est obtenue par traitement statistique d'un ensemble de représentations d'arcades historiques d'une population d'individus historiques ayant une ou plusieurs caractéristiques communes avec l'utilisateur, par exemple regroupant des personnes d'une classe d'âge identique à celle de l'utilisateur ou ayant été atteints par la même pathologie que l'utilisateur. Le traitement statistique peut par exemple consister à déterminer une représentation de référence qui minimise les différences de forme avec les représentations d'arcades historiques.

La représentation de référence peut être en particulier le modèle d'un typodont.

La représentation de référence peut être obtenue en agençant des modèles de dent historiques selon un agencement correspondant à une ou plusieurs images « d'analyse » de l'arcade de l'utilisateur, de préférence prises avec le téléphone portable de l'utilisateur. Il est en particulier possible de procéder suivant les étapes suivantes :
- création d'une bibliothèque historique comportant plus de 1 000, de préférence plus de 10 000 et/ou moins de 1 000 000 modèles de dent, dits « modèles de dent historiques », et de préférence attribution d'un numéro de dent à chaque modèle de dent historique ;
- analyse d'au moins une « image d'analyse » de l'arcade dentaire, par exemple au moyen d'un dispositif d'apprentissage profond, de préférence un réseau de neurones, de manière à déterminer au moins une zone de l'image d'analyse représentant une dent, ou « zone de dent d'analyse » et, de préférence le numéro de ladite dent ;
- pour chaque zone de dent d'analyse déterminée à l'étape précédente, recherche, dans la bibliothèque historique, d'un modèle de dent historique présentant une proximité de forme maximale avec la zone de dent d'analyse, ou « modèle de dent optimal » et, de préférence ayant le même numéro de dent que la dent représentée sur la zone de dent d'analyse ;
- agencement de l'ensemble des modèles de dents optimaux de manière à créer un modèle d'arcade dentaire qui présente une proximité maximale avec l'arcade représentée sur l'image d'analyse, ou « modèle assemblé » ;
- optionnellement, remplacement d'au moins un modèle de dent optimal par un autre modèle de dent et reprise à l'étape précédente de manière à maximiser la concordance entre le modèle assemblé et l'image d'analyse ;
- optionnellement, reprise de ladite analyse avec une autre image d'analyse et, à l'étape d'agencement et/ou de remplacement, recherche d'une concordance maximale avec l'ensemble des images d'analyse utilisées.

Un procédé pour générer une représentation de référence sous la forme d'un modèle en agençant des modèles de dent historiques est en particulier décrit dans la demande européenne n° 18 184486.

La représentation de référence peut être générée par l'appareil d'acquisition d'images, en particulier à partir d'images de l'arcade dentaire de l'utilisateur prises avec l'appareil d'acquisition d'images. Elle peut être générée par un autre appareil, par exemple par un ordinateur du professionnel de soins dentaires.

La représentation de référence peut être stockée dans une mémoire ou un cloud, accessible via des moyens de communication digitale. Elle est rendue accessible à l'ordinateur qui met en œuvre les étapes b) et c).

Après que la représentation de référence a été rendue disponible, le procédé comporte une pluralité de cycles d'étapes a) à d).

L'intervalle temporel entre deux cycles successifs est de préférence inférieur à 5 minutes, 1 minutes, 30 secondes, ou 1 seconde. De préférence, l'utilisateur acquiert des images actualisées en temps réel, de préférence en filmant son arcade dentaire, et des étapes b) à d) sont immédiatement réalisées pour chaque image actualisée. Le marquage, de préférence visuel, de la représentation de référence est ainsi réalisé en temps réel et l'utilisateur est guidé lors de la réalisation du film.

De préférence, un cycle est réalisé pour au moins une image actualisée prise face à l'utilisateur, au moins une image actualisée prise à droite de l'utilisateur et au moins une image actualisée prise à gauche de l'utilisateur.

De préférence, un cycle est réalisé pour au moins une image actualisée prise bouche ouverte et pour au moins une image actualisée prise bouche fermée.

Lors du premier cycle, la représentation de référence est partiellement marquée à l'étape b). Le marquage de représentation de référence est ensuite complété à chaque cycle suivant.

On décrit à présent en détail un exemple de cycle.

**A l'étape** a), on acquiert une image actualisée, de préférence une photo, représentant une arcade dentaire d'un utilisateur, au moyen d'un appareil d'acquisition d'images. De préférence, on acquiert un film avec l'appareil d'acquisition d'images, et l'image actualisée est extraite du film.

Dans un mode de réalisation particulier, une étape a) comporte l'acquisition d'une pluralité d'images actualisées, de préférence des photos, représentant une arcade dentaire d'un utilisateur, au moyen d'un appareil d'acquisition d'images, la pluralité d'images pouvant être extraite d'un film.

De préférence l'acquisition est réalisée par l'utilisateur lui-même.

L'image actualisée est de préférence « extraorale », c'est-à-dire sans que l'objectif de la caméra ou de l'appareil photo ne soit introduit dans la bouche de l'utilisateur.

L'appareil d'acquisition d'images peut être en particulier un téléphone portable, une tablette, un appareil photo ou un ordinateur, l'appareil d'acquisition d'images étant de préférence un téléphone portable ou une tablette, notamment pour que l'utilisateur puisse acquérir des images actualisées en tout lieu, et en particulier en dehors du cabinet d'un professionnel des soins dentaires, par exemple à plus de 1 km du cabinet d'un professionnel des soins dentaires.

Dans un mode de réalisation, l'utilisateur utilise un téléphone portable et un support sur lequel le téléphone portable est fixé de manière amovible, le support étant maintenu en appui sur l'utilisateur pendant l'acquisition de toutes les images actualisées. En particulier, le support peut être du type de ceux décrits dans PCT/EP2021/068702, EP17306361, PCT/EP2019/079565, PCT/EP2022/053847 ou FR2113577.

Dans un mode de réalisation préféré, l'utilisateur utilise un téléphone portable libre, c'est-à-dire dont il peut librement fixer la position et l'orientation, et en particulier non fixé à un support. Le procédé selon l'invention permet en effet de guider l'utilisateur dans ses prises de vue, de sorte qu'un guidage au moyen d'un support n'est pas indispensable. De préférence, l'appareil d'acquisition d'images n'est pas en contact avec la bouche de l'utilisateur, ni directement, ni par l'intermédiaire d'un support pour l'appareil d'acquisition d'images.

L'intervalle temporel entre l'étape préalable de génération de la représentation de référence et l'étape a) est de préférence inférieur à 4 semaines, 2 semaines, ou 1 semaine, en particulier lorsque ladite génération est réalisée à partir d'images d'analyse de l'arcade dentaire de l'utilisateur. L'étape préalable peut être également immédiatement avant l'étape a), par exemple moins de 5 minutes avant l'étape a).

**A l'étape b),** l'image actualisée est analysée de manière à identifier, dans l'image actualisée, une surface représentant, au moins partiellement, l'arcade dentaire de l'utilisateur, dite « surface 2D acquise ».

Lorsqu'une pluralité d'images actualisées est acquise à l'étape a) qui précède l'étape b), chaque image actualisée de la pluralité d'images actualisée est analysée de manière à identifier, dans chaque image actualisée, une surface 2D acquise.

L'analyse peut être faite au moyen de méthodes de segmentation classiques.

On recherche ensuite une partie de la surface de la représentation de référence qui correspond à la surface 2D acquise. De préférence, ladite partie résulte d'une relation univoque avec la surface 2D acquise, c'est-à-dire que, pour chaque surface 2D acquise, il est défini une unique partie de la surface de la représentation de référence.

De préférence, ladite partie est le résultat d'une projection de la surface 2D acquise sur la représentation de référence.

Dans un mode de réalisation préféré, la représentation de référence est un modèle. On recherche alors une vue du modèle en concordance avec l'image actualisée, c'est-à-dire qui représente l'arcade modélisée sensiblement comme l'image actualisée. Autrement dit, on recherche des conditions d'acquisition virtuelles adaptées pour une acquisition d'une vue du modèle présentant une concordance maximale avec l'image actualisée dans lesdites conditions d'acquisition virtuelles. Ces conditions d'acquisition virtuelles peuvent être déterminées en recherchant les conditions d'acquisition virtuelles permettant de superposer des points remarquables sur l'image actualisée à des points remarquables correspondant sur le modèle, de préférence au moyen d'un algorithme d'optimisation.

Une recherche d'une vue du modèle en concordance avec l'image actualisée est par exemple décrite dans décrit dans la demande européenne n° 18 184486.

Ladite vue et l'image actualisée peuvent être alors superposées sensiblement en registre l'une sur l'autre. La superposition en registre de la vue du modèle et de l'image actualisée permet de définir une région de la vue qui est couverte par, ou couvre la surface 2D acquise. Les limites de cette région peuvent être reportées sur le modèle. Elles délimitent alors la partie de la surface de la représentation de référence qui correspond à la surface 2D acquise. Ladite partie est alors le résultat d'une projection de la surface 2D acquise sur la représentation de référence suivant la direction d'observation du modèle selon laquelle la vue représente le modèle.

Ladite partie de la surface de la représentation de référence correspondant à la surface 2D acquise est alors marquée. On l'appelle « zone marquée élémentaire ». On appelle « zone marquée » l'union de l'ensemble des zones marquées élémentaires définies lors du cyclage. La zone marquée, initialement vide avant le premier cycle, comporte donc une zone marquée élémentaire à l'issue du premier cycle, puis se complète à chaque cycle, au fur et à mesure des déplacements de l'appareil d'acquisition et des acquisitions d'images actualisées. La « zone non marquée », représentant les parties de la représentation de référence qui correspondent à des régions de l'arcade dentaire qui n'apparaissent sur aucune image actualisée, se réduit donc progressivement.

**A l'étape c),** on détermine et on présente une information de guidage de l'appareil d'acquisition d'images.

L'information de guidage est une information utile pour aider l'utilisateur à déplacer l'appareil d'acquisition d'images afin que, lors du cycle suivant, l'image actualisée acquise représente une nouvelle surface 2D acquise qui permette de compléter la zone marquée, c'est-à-dire qui conduise à une zone marquée élémentaire au moins en partie à l'extérieur de la zone marquée définie à l'issue de l'étape b) du cycle courant.

L'information de guidage permet de renseigner l'utilisateur sur les parties de son arcade dentaire pour lesquelles il doit encore acquérir une ou plusieurs image(s) actualisée(s). Elle le guide pour qu'il oriente et/ou positionne l'appareil d'acquisition pour parvenir à l'acquisition souhaitée.

Dans un mode de réalisation, l'ordinateur mettant en œuvre l'étape c) détermine, par exemple par une recherche aléatoire ou avec un algorithme d'optimisation, lesdites nouvelles conditions d'acquisition, pour le cycle suivant, afin que la nouvelle surface 2D acquise dans ces nouvelles conditions d'acquisition maximise l'accroissement de la zone marquée de la représentation de référence. Ces nouvelles conditions d'acquisition peuvent être qualifiées de nouvelles conditions d'acquisition « cible ».

L'information de guidage peut comporter une information visuelle, et/ou une information sonore et/ou une information sensitive. Par exemple, l'information de guidage peut comporter des instructions audios indiquant à l'utilisateur de rapprocher ou d'éloigner l'appareil d'acquisition de ses dents, de décaler l'appareil d'acquisition vers la droite ou vers la gauche, de réaliser une rotation de l'appareil d'acquisition autour de l'arcade dentaire, d'ouvrir ou de fermer la bouche, ou d'ouvrir ou de fermer la mâchoire.

Une information sensitive peut être une vibration indiquant par exemple à l'utilisateur d'arrêter un mouvement.

De préférence, à l'étape c), on présente une information de guidage visuelle, de préférence sur un écran, de préférence sur un écran de l'appareil d'acquisition d'images, de préférence sur un écran de téléphone portable ou de tablette.

Dans un mode de réalisation, on affiche la zone marquée et, de préférence, la zone non marquée. Cet affichage permet de guider rapidement et efficacement l'utilisateur. Ce guidage, qui laisse une grande liberté à l'utilisateur, est intuitif, de sorte que l'utilisateur n'a pas besoin d'avoir été formé préalablement.

Le cyclage en temps réel permet avantageusement de visualiser immédiatement comment la zone marquée s'étend. La coloration progressive de la représentation de référence est ludique. Dans un mode de réalisation, un objectif peut être fixé à l'utilisateur, par exemple pour l'inciter à colorer la représentation de référence le plus rapidement possible, ce qui augmente encore l'aspect ludique de l'acquisition des images actualisées.

De préférence, la zone marquée est colorée, de préférence d'une couleur différence de la zone non marquée. De préférence, les contours des zones marquées élémentaires ne sont pas représentés spécifiquement, seul le contour de la zone marquée étant éventuellement représenté. Le marquage à l'étape b) est alors, de préférence, un marquage visuel consistant à étendre la zone marquée colorée de manière qu'elle incorpore la zone marquée élémentaire définie à ladite étape b).

Une coloration de la zone marquée, et de préférence un affichage de la zone non marquée, permettent d'indiquer indirectement à l'utilisateur comment déplacer l'appareil d'acquisition d'images de sorte à acquérir, lors du cycle suivant, une image actualisée représentant une partie de l'arcade dentaire correspondant à une zone non colorée et donc à une zone non marquée de la représentation de référence.

De manière équivalente à une coloration progressive de la zone marquée, il est possible d'effacer progressivement la zone non marquée. Sur l'écran, ces deux solutions sont similaires, l'effacement d'une zone étant sa coloration dans la couleur du fond d'écran.

L'information de guidage peut être déterminée en fonction des conditions d'acquisition de l'image actualisée acquise à l'étape a) précédente et de la zone non marquée.

La détermination de l'information de guidage en fonction de la zone non marquée, ou, de manière équivalente, de la zone marquée, implique que l'information de guidage n'est pas uniquement dépendante de la position de l'appareil d'acquisition d'images dans l'espace. Elle dépend de ce qu'a observé l'appareil d'acquisition d'images jusque-là, et donc non seulement de la position de l'appareil d'acquisition d'images dans l'espace, mais aussi de l'orientation de l'objectif de l'appareil d'acquisition d'images par rapport à l'arcade dentaire pendant l'acquisition de ces images.

Une représentation de la trajectoire de l'appareil d'acquisition d'images, comme décrit dans US2020193871A1, ne dépend pas de la zone marquée, mais seulement de la position de l'appareil d'acquisition d'images dans l'espace.

Les étapes b) et c) sont mises en œuvre par un programme d'ordinateur.

**A l'étape d),** l'utilisateur modifie la position et/ou l'orientation de l'appareil d'acquisition en fonction des informations de guidage.

De préférence, on répète le cycle des étapes a) à d) jusqu'à atteindre un critère d'arrêt.

Le critère d'arrêt peut être un rapport de l'aire de la surface marquée sur l'aire de la surface de la représentation de référence. En particulier, on peut répéter les étapes a) à d), jusqu'à ce que ledit rapport soit supérieur à 50%, de préférence supérieur à 75%, de préférence supérieur à 80%, de préférence supérieur à 90%, de préférence supérieur à 95%, de préférence soit de 100%, c'est-à-dire jusqu'à ce que la représentation de référence ne comporte plus de zone non marquée.

Le procédé de scannage selon l'invention comporte enfin, après arrêt du cyclage des a) à d), une étape de génération d'un modèle 3D à partir des images actualisées acquises au cours des différents cycles.

De préférence, à l'issue du cyclage des étapes a) à d), le procédé comporte une étape d'envoi des images actualisées acquises au cours des différents cycles et/ou d'un modèle généré à partir des images actualisées acquises au cours des différentes cycles, de préférence à un professionnel de soins dentaires.

Le modèle peut être en particulier utilisé pour qu'un professionnel de soins dentaires établisse un diagnostic, voire conçoive un plan de traitement orthodontique, voire fasse fabriquer un appareil orthodontique, de préférence une gouttière orthodontique.

### Dispositif

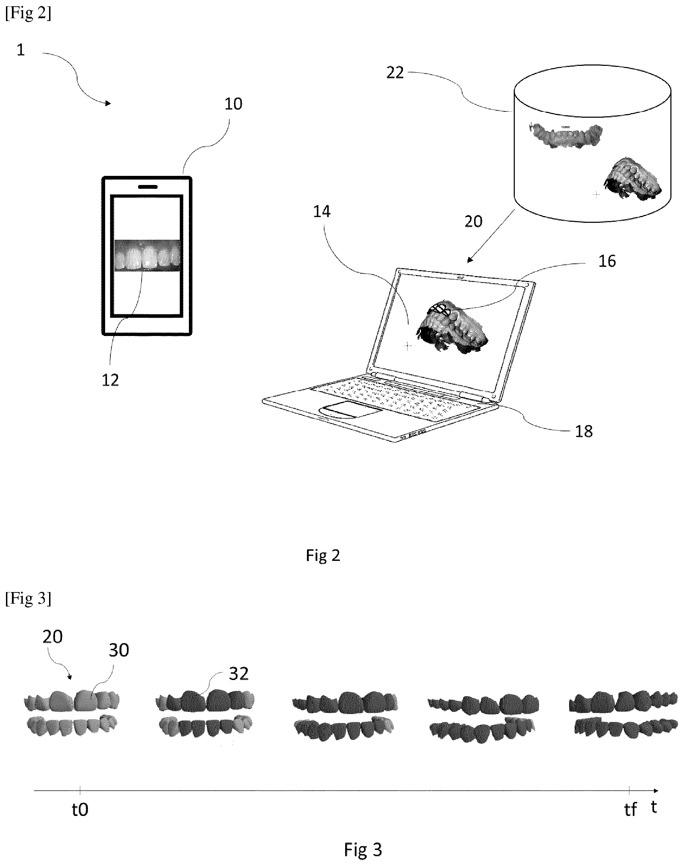

L'invention concerne également un dispositif pour mettre en œuvre un procédé de scannage selon l'invention. Un tel dispositif est schématisé sur la figure 2.

Un dispositif 1 selon l'invention comporte :
- optionnellement, un premier ordinateur comportant un premier programme d'ordinateur comprenant des instructions de code de programme pour générer la représentation de référence ;
- un appareil d'acquisition d'images 10 pour la mise en œuvre des étapes a) d'acquisition d'images actualisées 12,
- un écran 14 pour la présentation d'une information de guidage 16 lors des étapes c),
- un deuxième ordinateur 18 comportant un deuxième programme d'ordinateur comprenant des instructions de code de programme pour l'exécution des étapes b) et c), le deuxième ordinateur 18 recevant un modèle de référence 20 stocké dans une base de données 22,
- de préférence, un troisième ordinateur, identique ou différent du premier et/ou deuxième ordinateur(s), comportant un troisième programme d'ordinateur comprenant des instructions de code de programme pour générer un modèle final de l'arcade dentaire de l'utilisateur à partir des images actualisées ;
- de préférence, un quatrième ordinateur, identique ou différent du premier et/ou deuxième et/ou troisième ordinateur(s), comportant un quatrième programme d'ordinateur comprenant des instructions de code de programme pour comparer le modèle final à un autre modèle représentant l'arcade dentaire de l'utilisateur et/ou découper le modèle final en modèles de dent et, de préférence, déplacer lesdits modèles de dent.

De préférence, l'écran et/ou le premier ordinateur et/ou le deuxième ordinateur et/ou le troisième ordinateur et/ou le quatrième ordinateur est/sont intégré(s) à l'appareil d'acquisition d'images.

De préférence, l'appareil d'acquisition d'images est un téléphone portable ou une tablette comportant une caméra et un écran, le premier programme d'ordinateur et/ou le deuxième programme d'ordinateur et/ou le troisième programme d'ordinateur et/ou le quatrième programme d'ordinateur étant chargé(s) sur le téléphone portable ou la tablette, de préférence sous forme d'un logiciel spécialisé.

Le dispositif peut comporter des moyens de communication permettant l'envoi des images actualisées et/ou de la vidéo lorsque les images actualisées sont extraites d'une vidéo et/ou d'un modèle final généré à partir des images actualisées à l'issue des cycles d'étapes a) à d) et/ou permettant la réception de la représentation de référence avant le premier cycle.

Le dispositif peut comporter une mémoire permettant de stocker les images actualisées et/ou la vidéo lorsque les images actualisées sont extraites d'une vidéo et/ou d'un modèle final généré à partir des images actualisées.

### Exemple

Un utilisateur souhaite obtenir un modèle d'au moins une de ses arcades dentaires sans avoir à se déplacer chez un professionnel de soins dentaires, par exemple afin de faire vérifier l'état de sa dentition. Il lance un logiciel spécialisé, ou « application », sur son téléphone portable ou sa tablette, afin qu'il mette en œuvre le procédé selon l'invention.

On considère que le logiciel spécialisé met en œuvre une étape s'il la réalise elle-même ou s'il communique avec un ordinateur à distance à cet effet.

Lors de l'étape préalable, le logiciel spécialisé peut demander à l'utilisateur d'acquérir des images d'analyse, de préférence lui demande de prendre quelques photos ou un film, afin de déterminer des caractéristiques d'une de ses arcades dentaires, et choisir, dans une base de données, une représentation de référence sous la forme d'un modèle de référence représentant une arcade de référence présentant des caractéristiques identiques ou similaires. En particulier, une analyse des photos, éventuellement extraites du film, permet de déterminer la forme générale de l'arcade. Le modèle de référence peut être un modèle d'un typodont présentant par exemple la même largeur que celle de l'arcade de l'utilisateur.

Alternativement, le modèle de référence peut être un modèle d'un typodont standard utilisable pour une catégorie d'utilisateurs, voire un typodont universel, utilisable quel que soit l'utilisateur.

Le logiciel spécialisé affiche alors le modèle de référence sur l'écran du téléphone portable et demande à l'utilisateur de filmer son arcade.

A l'étape a), en mode selfie et de préférence sans utilisation d'un support, l'utilisateur filme son arcade. De préférence, il utilise cependant un écarteur, et en particulier pour écarter les commissures des lèvres afin de filmer les dents au fond de sa bouche et/ou pour écarter les lèvres supérieure et inférieure afin de bien dégager les incisives, de préférence jusqu'à faire apparaitre la gencive qui les ceinture. Les images actualisées sont des photos extraites du film, de préférence à intervalle de temps régulier.

A l'étape b), chaque image actualisée du film est analysée par le logiciel spécialisé afin d'identifier si elle représente une partie de l'arcade. L'analyse permet, le cas échéant, d'identifier une partie de l'image actualisée, éventuellement en plusieurs morceaux, qui représente l'arcade dentaire, c'est-à-dire d'identifier une « surface 2D acquise ».

La surface 2D acquise est alors projetée sur le modèle de référence. A cet effet, le logiciel spécialisé détermine une direction qui permet d'observer le modèle de référence afin d'en avoir une vue sensiblement identique à la représentation de l'arcade de l'utilisateur sur l'image actualisée. La surface 2D acquise est alors projetée sur le modèle de référence suivant cette direction, ce qui permet d'identifier une partie de la surface du modèle de référence qui représente les mêmes parties de l'arcade de référence que celles de l'arcade de l'utilisateur représentées par la surface 2D acquise.

Cette partie de la surface du modèle de référence, ou « zone élémentaire marquée », est alors ajoutée à la zone marquée du modèle de référence, qui est l'union l'ensemble des zones élémentaires marquées lors des cycles antérieurs. A tout instant, le logiciel spécialisé peut ainsi indiquer la zone non marquée, pour laquelle au moins une image actualisée doit encore être prise et analysée.

Dans cet exemple, on considère que le but est de couvrir tout le modèle de référence, c'est-à-dire que le but est que la zone marquée soit constituée de toute la surface du modèle de référence, comme illustré figure 3 à l'instant t_{f}. Dans un mode de réalisation, le but du procédé n'est cependant de couvrir qu'une portion de la surface du modèle de référence, et plus généralement de la représentation de référence. Cette portion peut être rendue identifiable sur le modèle de référence, par exemple en en traçant le contour. Alternativement, seule ladite portion peut être affichée sur l'écran de téléphone portable ou de la tablette.

Le logiciel spécialisé fournit alors une information de guidage afin que l'utilisateur dispose la caméra du téléphone portable ou de la tablette pour atteindre le but.

Dans un mode de réalisation préféré, le logiciel spécialisé marque visuellement la zone marquée, de préférence en la colorant différemment de la zone non marquée. L'utilisateur voit ainsi immédiatement la zone non marquée et positionne en conséquence la caméra. La coloration étant en temps réel, l'utilisateur reçoit ainsi immédiatement un retour sur la pertinence du positionnement instantané de la caméra et peut l'adapter en conséquence.

La figure 3 illustre différentes vues d'un modèle de référence comportant une marque visuelle constituant l'information de guidage au cours du temps t, le modèle de référence 20 étant composé de modèles de dents 30 étant colorés progressivement en fonction des acquisitions d'images réalisées au cours de la mise en œuvre de cycles d'étapes a) à d).

Avant le premier cycle, à l'instant t₀, la zone marquée du modèle de référence est l'ensemble vide.

Chaque modèle de dent 30 peut être coloré lorsque plus de 80% de la surface du modèle de dent est couverte par la zone marquée du modèle de référence, de préférence lorsque plus de 90% de la surface du modèle de dent est couverte par la zone marquée du modèle de référence, encore mieux lorsque toute la surface du modèle de dent est couverte par la zone marquée du modèle de référence. La présentation des modèles de dents colorés 32 fournissant une information de guidage à l'utilisateur.

Un modèle de dent peut être coloré partiellement, la coloration correspondant aux surfaces du modèle de dent couverte par la zone marquée du modèle de référence. Ainsi, l'utilisateur peut facilement orienter l'appareil d'acquisition d'images de sorte à acquérir une surface du modèle de dent non couverte par la zone marquée du modèle de référence. D'autres informations de guidage peuvent être fournies, alternativement ou, de préférence, en complément du marquage visuel.

Dans un mode de réalisation, le logiciel spécialisé détermine de nouvelles conditions d'acquisition « cible » de la caméra, c'est-à-dire par exemple qui conduiraient à la plus grande surface 2D acquise ou à la plus grande zone marquée élémentaire, c'est-à-dire des nouvelles conditions d'acquisition particulièrement efficaces pour couvrir le plus rapidement possible la surface du modèle de référence. De préférence, le logiciel spécialisé guide vers les nouvelles conditions d'acquisition cible, par exemple en colorant différemment une région du modèle de référence, ou en affichant une flèche indiquant un mouvement souhaité pour la caméra, ou en émettant un signal sonore d'autant plus fort, ou d'autant plus aigu que la caméra approche des nouvelles conditions d'acquisition cible.

Quand le but est atteint, par exemple parce que toute la surface du modèle de référence est couverte par la zone marquée, le critère d'arrêt est atteint. Un message peut être envoyé à l'utilisateur pour l'en informer, afin qu'il mette fin au film.

Le logiciel spécialisé dispose alors de suffisamment d'images actualisées pour générer un modèle « actualisé » de l'arcade de l'utilisateur. Le modèle final peut être généré directement à partir des images actualisées, à la manière d'un scan 3D. Il peut résulter de la déformation d'un autre modèle, par exemple du modèle de référence, la déformation étant guidée pour que le modèle final corresponde le mieux possible aux images actualisées.

Dans l'exemple de la figure 3, à l'instant t_{f} l'ensemble des modèles de dents sont colorés indiquant à l'utilisateur que le dernier cycle d'étapes a) à d) a été effectué et que l'étape finale de génération d'un modèle tridimensionnel final à partir des images actualisées peut être réalisée.

Comme il apparait clairement à présent, un dispositif et un procédé selon l'invention permettent avantageusement d'augmenter l'autonomie de l'utilisateur et d'améliorer la qualité et le contenu des images acquises par un utilisateur n'ayant aucune connaissance particulière dans le domaine dentaire. Ils permettent avantageusement de réaliser un modèle 3D d'une arcade dentaire d'un utilisateur, à distance. Ils facilitent ainsi grandement la détermination à distance d'un traitement orthodontique, sans que l'utilisateur ait besoin de prendre rendez-vous chez un professionnel de soins dentaires.

Bien entendu, l'invention n'est pas limitée aux modes de réalisation décrits ci-dessus et représentés.

En particulier, l'invention n'est pas limitée par l'utilisation du modèle final. Ce dernier peut par exemple servir à l'établissement d'un diagnostic, ou d'un plan de traitement orthodontique, ou à la fabrication d'une gouttière orthodontique. En particulier, le modèle final peut représenter seulement une zone d'intérêt pour une analyse de la situation dentaire de l'utilisateur ou pour déterminer un traitement orthodontique, par exemple une zone dont on veut évaluer l'hygiène, un risque de récidive dans une zone particulière de l'arcade dentaire de l'utilisateur, une zone pour laquelle l'utilisateur et/ou un professionnel de soins dentaires souhaite programmer un éventuel traitement esthétique.

La représentation de référence n'est pas limitée à un modèle de référence. Elle peut être une image bidimensionnelle, même si un modèle est préféré.

Dans un mode de réalisation particulier de l'invention, l'étape d) du procédé de scannage est optionnelle, le procédé de scannage comportant :
- une étape préalable de détermination d'une représentation d'une arcade dentaire de référence, dite « représentation de référence », puis
- une pluralité de cycles d'étapes a) à c) suivantes :
   a) acquisition, dans des conditions d'acquisition, d'une image bidimensionnelle de ladite arcade dentaire, ou « image actualisée », au moyen d'un appareil d'acquisition d'images, par l'utilisateur ;
   b) analyse de l'image actualisée de manière à identifier la surface de l'image actualisée représentant l'arcade dentaire de l'utilisateur, dite « surface 2D acquise », puis délimitation et marquage d'une surface de la représentation de référence correspondant à la surface 2D acquise, ou « zone marquée élémentaire », et ajout de la zone marquée élémentaire dans une « zone marquée de la représentation de référence » regroupant l'ensemble, vide avant le premier cycle, des zones marquées élémentaires définies lors des cycles précédents ;
   c) détermination et présentation à l'utilisateur d'une information de guidage de l'appareil d'acquisition vers de nouvelles conditions d'acquisition adaptées pour que le cycle suivant augmente la zone marquée de la représentation de référence ;
   d) de préférence, déplacement de l'appareil d'acquisition vers lesdites nouvelles conditions d'acquisition ;
le procédé comportant, après le dernier cycle, une étape finale de génération d'un modèle tridimensionnel « final » à partir desdites images actualisées.

Bien entendu, le procédé peut encore notamment comporter une ou plusieurs des caractéristiques optionnelles décrites précédemment et qui sont compatibles avec une étape d) optionnelle.

## Revendications

1. Procédé de scannage d'une arcade dentaire d'un utilisateur, le procédé comportant
- une étape préalable de détermination d'une représentation d'une arcade dentaire de référence, dite « représentation de référence », puis
- une pluralité de cycles d'étapes a) à c), de préférence a) à d) suivantes :
a) acquisition, dans des conditions d'acquisition, d'une image bidimensionnelle de ladite arcade dentaire, ou « image actualisée », au moyen d'un appareil d'acquisition d'images (10), par l'utilisateur ;
b) analyse de l'image actualisée de manière à identifier la surface de l'image actualisée représentant l'arcade dentaire de l'utilisateur, dite « surface 2D acquise », puis délimitation et marquage d'une surface de la représentation de référence correspondant à la surface 2D acquise, ou « zone marquée élémentaire », et ajout de la zone marquée élémentaire dans une « zone marquée de la représentation de référence » regroupant l'ensemble, vide avant le premier cycle, des zones marquées élémentaires définies lors des cycles précédents ;
c) détermination et présentation à l'utilisateur d'une information de guidage (16) de l'appareil d'acquisition vers de nouvelles conditions d'acquisition adaptées pour que le cycle suivant augmente la zone marquée de la représentation de référence ;
d) de préférence, déplacement de l'appareil d'acquisition vers lesdites nouvelles conditions d'acquisition ;
le procédé comportant, après le dernier cycle, une étape finale de génération d'un modèle tridimensionnel « final » à partir desdites images actualisées (12).

2. Procédé selon la revendication 1, dans lequel les images actualisées des cycles successifs sont extraites d'un film acquis par l'utilisateur par une tablette ou un téléphone portable, lesdits cycles étant exécutés en temps réel pendant l'acquisition dudit film, au moins une partie de l'information de guidage étant présentée sur un écran de ladite tablette ou dudit téléphone, respectivement.

3. Procédé selon l'une quelconque des revendications précédentes, dans lequel l'information de guidage comporte une présentation de la zone marquée sur un écran de l'appareil d'acquisition d'images en superposition avec la représentation de référence dans laquelle la zone non marquée présente une apparence différente de la zone marquée.

4. Procédé selon l'une quelconque des revendications précédentes, dans lequel la représentation de référence est :
une ou plusieurs illustrations, en 3D ou 2D, d'une arcade dentaire, éventuellement photoréaliste, ou un modèle « de référence » d'une arcade « de référence »
- représentative des arcades « historiques » d'un ensemble comportant plus de 10 individus historiques, ou
- constituée par assemblage de modèles de dents suivant un agencement déterminé par analyse d'une ou plusieurs images d'analyse de l'arcade dentaire de l'utilisateur.

5. Procédé selon la revendication immédiatement précédente, dans laquelle, à l'étape c) d'un cycle quelconque, on présente une vue du modèle de référence observé suivant une direction d'observation orientée, par rapport au modèle de référence, sensiblement comme la direction d'acquisition de l'image actualisée à l'étape a) dudit cycle, par rapport à l'arcade dentaire de l'utilisateur, de sorte que ladite vue est sensiblement superposable en registre avec ladite image actualisée.

6. Procédé selon l'une quelconque des revendications précédentes, dans lequel, à une étape c) quelconque, l'appareil d'acquisition d'images fournit une information variable en fonction d'une différence entre les conditions d'acquisition réelles et lesdites nouvelles conditions d'acquisition.

7. Procédé selon l'une quelconque des revendications précédentes, dans lequel, pour l'étape a) d'au moins un cycle, on écarte les lèvres et/ou les joues de l'arcade dentaire de l'utilisateur de manière à exposer à l'appareil d'acquisition d'images au moins une dent.

8. Procédé selon l'une quelconque des revendications précédentes, dans lequel, lors du cyclage, on acquiert
- au moins une image actualisée prise face à l'utilisateur et au moins une image actualisée prise à droite de l'utilisateur et au moins une image actualisée prise à gauche de l'utilisateur ; et/ou
au moins une image actualisée prise face à l'utilisateur et au moins une image actualisée prise de haut par rapport à l'utilisateur et au moins une image actualisée prise de bas par rapport à l'utilisateur ; et/ou
- au moins une image actualisée prise bouche ouverte et au moins une image actualisée prise bouche fermée.

9. Procédé selon l'une quelconque des revendications précédentes, dans lequel l'utilisateur est à plus de 100 m de tout professionnel de soins dentaires.

10. Procédé selon l'une quelconque des revendications précédentes, dans lequel on arrête le cyclage quand plus de 50% de la surface de la représentation de référence sont représentés sur au moins une image actualisée.

11. Procédé selon l'une quelconque des revendications précédentes, dans lequel on compare le modèle final à un autre modèle représentant l'arcade dentaire de l'utilisateur et/ou on compare le modèle final au modèle de référence, de préférence on présente cette comparaison à l'utilisateur, par exemple en superposant, de préférence en registre, le modèle final et le modèle de référence.

12. Procédé selon l'une quelconque des revendications précédentes, dans lequel l'information de guidage comporte une information sonore, une information visuelle, et/ou une information sensitive.

13. Procédé selon l'une quelconque des revendications précédentes, dans lequel la représentation de référence est un modèle et on détermine la surface de la représentation de référence correspondant à la surface 2D acquise
- en recherchant des conditions d'acquisition virtuelles adaptées pour une acquisition d'une vue de la représentation de référence, dite « image de référence », présentant une concordance maximale avec l'image actualisée dans lesdites conditions d'acquisition virtuelles ; puis
- en projetant la surface 2D acquise, sur la représentation de référence et suivant la direction d'observation de ladite représentation de référence dans lesdites conditions d'acquisition virtuelles.

14. Procédé selon la revendication précédente, dans lequel les conditions d'acquisition virtuelles sont déterminées en cherchant à superposer des points remarquables de l'arcade représentée sur l'image actualisée avec les points remarquables correspondants de la représentation de référence.

15. Dispositif pour la mise en œuvre un procédé selon l'une quelconque des revendications précédentes, comportant
- optionnellement, un premier ordinateur comportant un premier programme d'ordinateur comprenant des instructions de code de programme pour générer la représentation de référence ;
- un appareil d'acquisition d'images (10) pour la mise en œuvre des étapes a) :
- de préférence, un écran (14) pour la présentation d'une information de guidage (16) lors des étapes c) ;
- un deuxième ordinateur (18), identique ou différent du premier ordinateur, comportant un deuxième programme d'ordinateur comprenant des instructions de code de programme pour l'exécution des étapes b) et c) ;
- un troisième ordinateur, identique ou différent du premier et/ou deuxième ordinateur(s), comportant un troisième programme d'ordinateur comprenant des instructions de code de programme pour la mise en œuvre de l'étape finale de génération du modèle final de l'arcade dentaire de l'utilisateur à partir des images actualisées (12);
- de préférence, un quatrième ordinateur, identique ou différent du premier et/ou deuxième et/ou troisième ordinateur(s), comportant un quatrième programme d'ordinateur comprenant des instructions de code de programme pour comparer le modèle final à un autre modèle représentant l'arcade dentaire de l'utilisateur.

## Patentansprüche

1. Verfahren zum Scannen eines Zahnbogens eines Benutzers, wobei das Verfahren Folgendes aufweist:
- einen vorausgehenden Schritt des Bestimmens einer als "Referenzabbildung" bezeichneten Abbildung eines Referenzzahnbogens und anschließend
- eine Vielzahl von Zyklen der folgenden Schritte a) bis c), vorzugsweise a) bis d):
a) Erfassen eines zweidimensionalen Bildes des Zahnbogens bzw. "aktualisierten Bildes" mittels einer Bilderfassungseinrichtung (10) durch den Benutzer unter Erfassungsbedingungen;
b) Analysieren des aktualisierten Bildes, um die als "erfasste 2D-Oberfläche" bezeichnete Oberfläche des aktualisierten Bildes, die den Zahnbogen des Benutzers darstellt, zu identifizieren und anschließend Begrenzen und Markieren einer Oberfläche der Referenzabbildung, die der erfassten 2D-Oberfläche entspricht, bzw. des "elementaren markierten Bereichs" und Hinzufügen des elementaren markierten Bereichs zu einem "markierten Bereich der Referenzabbildung", in dem die vor dem ersten Zyklus leere Zusammenstellung der in den vorstehenden Zyklen definierten elementaren markierten Bereiche zusammengefasst ist;
c) Bestimmen und Darstellen von Führungsinformation (16) für den Benutzer zum Führen der Erfassungseinrichtung zu neuen Erfassungsbedingungen, die so ausgelegt sind, dass der folgende Zyklus den markierten Bereich der Referenzabbildung vergrößert;
d) vorzugsweise Bewegen der Erfassungseinrichtung zu den neuen Erfassungsbedingungen;
wobei das Verfahren nach dem letzten Zyklus einen abschließenden Schritt zur Erzeugung eines "finalen" dreidimensionalen Modells aus den aktualisierten Bildern (12) umfasst.

2. Verfahren nach Anspruch 1, wobei die aktualisierten Bilder von aufeinanderfolgenden Zyklen aus einem Film extrahiert werden, der durch den Benutzer mittels eines Tablets oder eines Mobiltelefons erfasst wird, wobei die Zyklen in Echtzeit während der Erfassung des Films ausgeführt werden, wobei mindestens ein Teil der Führungsinformationen auf einem Bildschirm des Tablets bzw. Telefons dargestellt wird.

3. Verfahren nach einem der vorstehenden Ansprüche, wobei die Führungsinformationen eine Anzeige des markierten Bereichs auf einem Bildschirm der Bilderfassungseinrichtung als Überblendung auf die Referenzabbildung umfassen, wobei der nicht markierte Bereich ein anderes Erscheinungsbild als der markierte Bereich aufweist.

4. Verfahren nach einem der vorstehenden Ansprüche, wobei die Referenzabbildung Folgendes ist:
eine oder mehrere gegebenenfalls fotorealistische Darstellungen eines Zahnbogens bzw. ein "Referenz"-Modell eines "Referenz"-Zahnbogens in 3D oder 2D, die
- für die "historischen" Bögen einer Zusammenstellung von mehr als 10 historischen Individuen repräsentativ sind oder
- durch Zusammenfügen von Zahnmodellen gemäß einer durch Analyse eines oder mehrerer Analysebilder des Zahnbogens des Benutzers bestimmten Anordnung gebildet werden.

5. Verfahren nach dem unmittelbar vorstehenden Anspruch, wobei in Schritt c) eines beliebigen Zyklus eine Ansicht des in einer Betrachtungsrichtung betrachteten Referenzmodells angezeigt wird, die in Bezug auf das Referenzmodell im Wesentlichen wie die Erfassungsrichtung des in Schritt a) des Zyklus aktualisierten Bildes in Bezug auf den Zahnbogen des Benutzers ausgerichtet ist, sodass die Ansicht im Wesentlichen deckungsgleich mit dem aktualisierten Bild überblendbar ist.

6. Verfahren nach einem der vorstehenden Ansprüche, wobei in einem beliebigen Schritt c) die Bilderfassungseinrichtung in Abhängigkeit von einer Differenz zwischen den tatsächlichen Erfassungsbedingungen und den neuen Erfassungsbedingungen variable Informationen bereitstellt.

7. Verfahren nach einem der vorstehenden Ansprüche, wobei für Schritt a) mindestens eines Zyklus die Lippen und/oder die Wangen vom Zahnbogen des Benutzers derart weggezogen werden, dass für die Bilderfassungseinrichtung mindestens ein Zahn freigelegt wird.

8. Verfahren nach einem der vorstehenden Ansprüche, wobei während des Durchlaufens der Zyklen Folgendes erfasst wird:
- mindestens ein zum Benutzer frontal aufgenommenes aktualisiertes Bild und mindestens ein zum Benutzer rechts aufgenommenes aktualisiertes Bild und mindestens ein zum Benutzer links aufgenommenes aktualisiertes Bild; und/oder
- mindestens ein zum Benutzer frontal aufgenommenes aktualisiertes Bild und mindestens ein zum Benutzer von oben aufgenommenes aktualisiertes Bild und mindestens ein zum Benutzer von unten aufgenommenes aktualisiertes Bild; und/oder
- mindestens ein bei geöffnetem Mund aufgenommenes aktualisiertes Bild und mindestens ein bei geschlossenem Mund aufgenommenes aktualisiertes Bild.

9. Verfahren nach einem der vorstehenden Ansprüche, wobei der Benutzer mehr als 100 m von jedweder zahnmedizinischen Fachkraft entfernt ist.

10. Verfahren nach einem der vorstehenden Ansprüche, wobei das Durchlaufen der Zyklen beendet wird, wenn mehr als 50 % der Oberfläche der Referenzabbildung auf mindestens einem aktualisierten Bild dargestellt sind.

11. Verfahren nach einem der vorstehenden Ansprüche, wobei das finale Modell mit einem anderen Modell verglichen wird, das den Zahnbogen des Benutzers darstellt, und/oder das finale Modell mit dem Referenzmodell verglichen wird, wobei dieser Vergleich vorzugsweise dem Benutzer zum Beispiel durch vorzugsweise deckungsgleiches Überblenden des finalen Modells und des Referenzmodells angezeigt wird.

12. Verfahren nach einem der vorstehenden Ansprüche, wobei die Führungsinformationen akustische Informationen, visuelle Informationen und/oder sensorische Informationen umfassen.

13. Verfahren nach einem der vorstehenden Ansprüche, wobei die Referenzabbildung ein Modell ist und die der erfassten 2D-Oberfläche entsprechende Oberfläche der Referenzabbildung bestimmt wird
- durch Suchen nach geeigneten virtuellen Erfassungsbedingungen, die für eine Erfassung einer Ansicht der als "Referenzbild" bezeichneten Referenzabbildung, die unter den virtuellen Erfassungsbedingungen eine maximale Übereinstimmung mit dem aktualisierten Bild aufweist, ausgelegt sind; und anschließend
- durch Projizieren der erfassten 2D-Oberfläche auf die Referenzabbildung und entlang der Betrachtungsrichtung der Referenzabbildung unter den virtuellen Erfassungsbedingungen.

14. Verfahren nach dem vorstehenden Anspruch, wobei die virtuellen Erfassungsbedingungen bestimmt werden, indem versucht wird, markante Punkte des auf dem aktualisierten Bild dargestellten Bogens über die entsprechenden markanten Punkten der Referenzabbildung zu legen.

15. Vorrichtung zum Durchführen eines Verfahrens nach einem der vorstehenden Ansprüche, umfassend
- optional einen ersten Computer, der ein erstes Computerprogramm aufweist, das Programmcodeanweisungen zum Generieren der Referenzabbildung umfasst;
- eine Bilderfassungseinrichtung (10) zum Durchführen der Schritte a);
- vorzugsweise einen Bildschirm (14) zum Anzeigen von Führungsinformationen (16) während der Schritte c);
- einen zweiten Computer (18), der mit dem ersten Computer identisch oder von diesem verschieden ist, der ein zweites Computerprogramm aufweist, das Programmcodeanweisungen zum Ausführen der Schritte b) und c) umfasst;
- einen dritten Computer, der mit dem ersten und/oder zweiten Computer identisch oder davon verschieden ist, der ein drittes Computerprogramm aufweist, das Programmcodeanweisungen zum Durchführen des finalen Schritts des Generierens des finalen Modells des Zahnbogens des Benutzers aus den aktualisierten Bildern (12) umfasst;
- vorzugsweise einen vierten Computer, der mit dem ersten und/oder zweiten und/oder dritten Computer identisch oder davon verschieden ist, der ein viertes Computerprogramm aufweist, das Programmcodeanweisungen umfasst, um das finale Modell mit einem anderen Modell, das den Zahnbogen des Benutzers darstellt, zu vergleichen.

## Claims

1. Method for scanning a dental arch of a user, the method comprising
- a prior step of determining a representation of a reference dental arch, referred to as a "reference representation", then
- a plurality of cycles of the following steps a) to c), preferably steps a) to d):
a) acquiring, by the user, under acquisition conditions, a two-dimensional image of said dental arch, or "updated image", by means of an image acquisition apparatus (10);
b) analyzing the updated image so as to identify the surface of the updated image representing the dental arch of the user, referred to as the "acquired 2D surface", then outlining and labeling a surface of the reference representation that corresponds to the acquired 2D surface, or "elementary labeled region", and adding the elementary labeled region to a "labeled region of the reference representation" that brings together all the elementary labeled regions defined during the previous cycles, which were empty prior to the first cycle;
c) determining and displaying to the user guide information (16) for guiding the acquisition apparatus toward new acquisition conditions suitable for the next cycle to increase the labeled region of the reference representation;
d) preferably, moving the acquisition apparatus toward said new acquisition conditions;
the method comprising, after the last cycle, a final step of generating a "final" three-dimensional model from said updated images (12).

2. Method according to claim 1, wherein the updated images of successive cycles are extracted from a video acquired by the user via a tablet or a cell phone, said cycles being executed in real time during the acquisition of said video, at least part of the guide information being displayed on a screen of said tablet or said phone, respectively.

3. Method according to any one of the preceding claims, wherein the guide information comprises a display of the labeled region on a screen of the image acquisition apparatus superimposed on the reference representation, wherein the unlabeled region has an appearance that is different from the labeled region.

4. Method according to any one of the preceding claims, wherein the reference representation is:
one or more 3D or 2D illustrations of a dental arch, optionally photorealistic illustrations, or a "reference" model of a "reference" arch
- representative of the "historic" arches of a set comprising more than 10 historic individuals, or
- formed by assembling tooth models in an arrangement determined by analysis of one or more analysis images of the dental arch of the user.

5. Method according to the immediately preceding claim, wherein, in step c) of any cycle, a view is displayed of the reference model observed along a direction of observation that is oriented, with respect to the reference model, substantially like the direction of acquisition of the image updated in step a) of said cycle, with respect to the dental arch of the user, so that said view is substantially superimposable in alignment with said updated image.

6. Method according to any one of the preceding claims, wherein, in any step c), the image acquisition apparatus provides variable information depending on a difference between the actual acquisition conditions and said new acquisition conditions.

7. Method according to any one of the preceding claims, wherein, for step a) of at least one cycle, the lips and/or cheeks are moved away from the dental arch of the user so as to expose at least one tooth to the image acquisition apparatus.

8. Method according to any one of the preceding claims, wherein, during the cycles, the following are acquired:
- at least one updated image taken facing the user, at least one updated image taken to the right of the user and at least one updated image taken to the left of the user; and/or
at least one updated image taken facing the user, at least one updated image taken from above with respect to the user and at least one updated image taken from below with respect to the user; and/or
- at least one updated image taken with the mouth open and at least one updated image taken with the mouth closed.

9. Method according to any one of the preceding claims, wherein the user is more than 100 m from any dental care professional.

10. Method according to any one of the preceding claims, wherein the cycles are stopped when more than 50% of the surface of the reference representation is represented on at least one updated image.

11. Method according to any one of the preceding claims, wherein the final model is compared with another model representing the dental arch of the user and/or the final model is compared with the reference model, this comparison is preferably displayed to the user, for example by superimposing, preferably in alignment, the final model and the reference model.

12. Method according to any one of the preceding claims, wherein the guide information comprises sound information, visual information, and/or sensitive information.

13. Method according to any one of the preceding claims, wherein the reference representation is a model and the surface of the reference representation that corresponds to the acquired 2D surface is determined
- by searching for suitable virtual acquisition conditions for acquiring a view of the reference representation, referred to as the "reference image", that best matches the image updated under said virtual acquisition conditions; then
- by projecting the acquired 2D surface onto the reference representation along the direction of observation of said reference representation under said virtual acquisition conditions.

14. Method according to the preceding claim, wherein the virtual acquisition conditions are determined by seeking to superimpose noteworthy points of the arch represented in the updated image on the corresponding noteworthy points of the reference representation.

15. Device for carrying out a method according to any one of the preceding claims, comprising
- optionally, a first computer comprising a first computer program comprising program code instructions for generating the reference representation;
- an image acquisition apparatus (10) for carrying out steps a);
- preferably, a screen (14) for displaying guide information (16) during steps c);
- a second computer (18), identical to or different from the first computer, comprising a second computer program comprising program code instructions for executing steps b) and c);
- a third computer, identical to or different from the first and/or second computer(s), comprising a third computer program comprising program code instructions for carrying out the final step of generating the final model of the dental arch of the user from the updated images (12);
- preferably, a fourth computer, identical to or different from the first and/or second and/or third computer(s), comprising a fourth computer program comprising program code instructions for comparing the final model with another model representing the dental arch of the user.
